# FASCICULE DE BREVET EUROPEEN

(11) **EP 3 962 509 B1**
(45) Date de publication et mention de la délivrance du brevet: **26.06.2024**
(21) Numéro de dépôt: 20721618.5
(22) Date de dépôt: 30.04.2020
(51) Int. Cl.: A61K 36/282, A61K 36/53, A61K 36/899, A61K 36/48, A61K 9/00, A61P 33/14, A23K 10/30, A23K 50/40, A23K 50/42, A61K 47/46

(54) **COMPOSITION CONTRE LES ECTOPARASITES**
ZUSAMMENSETZUNG GEGEN EKTOPARASITEN
COMPOSITION AGAINST ECTOPARASITES

(30) Priorité: 30.04.2019 FR 1904596
(43) Date de publication de la demande: 09.03.2022
(73) Titulaire: Biodevas Laboratoires, 72460 Savigne-L'Evêque (FR)
(72) Inventeur: BLUA, François, 72000 LE MANS (FR); BLUA, Jean Louis, 72460 SAVIGNE L'EVÊQUE (FR)
(74) Mandataire: LLR
(86) Numéro de dépôt international: PCT/EP2020/062050
(87) Numéro de publication internationale: WO 2020/221867

(56) Documents cités:
- WO-A1-2013/184172
- WO-A2-2009/117623
- FR-A1- 2 541 898
- FR-A1- 3 066 392
- US-A1- 2005 008 656
- US-A1- 2005 207 982
- US-A1- 2008 193 387
- US-A1- 2015 374 624
- LANS CHERYL ET AL: "Medicinal plant treatments for fleas and ear problems of cats and dogs in British Columbia, Canada", PARASITOLOGY RESEARCH, vol. 103, no. 4, septembre 2008 (2008-09), pages 889-898, XP019630818, ISSN: 0932-0113
- DATABASE GNPD [Online] MINTEL; 16 février 2018 (2018-02-16), anonymous: "Pastis", XP055655911, extrait de www.gnpd.com Database accession no. 5458449

## Description

L'invention concerne une composition anti-ectoparasites, notamment une composition destinée à protéger des et / ou éliminer les parasites externes des animaux de compagnie.

Dans toutes les études épidémiologiques, l'infestation par les puces (la pulicose) est incontestablement au premier rang des causes de désagrément chez les carnivores domestiques. Les puces sont des insectes piqueurs dont les adultes vivent sur les mammifères et les oiseaux. En France, l'espèce de puces la plus couramment rencontrée chez nos carnivores domestiques est *Ctenocephalides felis.* Plus rarement, il est possible de retrouver sur les chats et les chiens des puces de hérissons, d'oiseaux, de lapins et même parfois la puce de l'homme.

Les puces, une fois adultes ne peuvent vivre que sur un animal, alors que les oeufs et les larves se développent dans le milieu extérieur.

Une fois sur leur hôte, les adultes se nourrissent de sang en perçant la peau de l'animal qui les héberge. Leur espérance de vie est en théorie de plusieurs mois, mais elles sont souvent éliminées au bout d'une à trois semaines lorsque le chien ou le chat se toilette ou se mordille.

Le mode de vie des puces est dépendant des conditions environnementales. Quand elles sont idéales (humidité, chaleur, hôtes à proximité), l'ensemble des étapes de développement peut être accompli en moins de 2 semaines ! Une seule puce femelle peut ainsi produire plusieurs dizaines de milliers de nouveaux adultes en un mois. Le nombre de puces sur les animaux et surtout dans l'environnement peut devenir très rapidement considérable

Comme la chaleur est nécessaire à la survie des larves de puces, elles peuvent se développer à l'extérieur du printemps à l'automne, mais pas en hiver. Cependant, le chauffage des maisons et appartements leur permet de se développer à l'intérieur tout au long de l'année.

Il est important de prendre conscience que la contamination d'un animal par les puces se fait surtout à partir des adultes pré-émergeants présents dans l'environnement, et que cette contamination peut venir aussi bien de l'extérieur (sauf en hiver) que de l'intérieur de la maison. La contamination par passage d'une puce adulte d'un animal à un autre est certes possible, mais finalement plutôt rare. Elle nécessite un contact étroit entre les deux animaux.

L'infestation par des puces est dénommée pulicose. La présence de puces sur un animal présente de nombreux inconvénients pour celui-ci :
- Des douleurs ressenties par l'animal lors de la piqure,
- Une démangeaison,
- Une perte sanguine en cas de sur infestation, et
- Le risque de transmission de maladies. Les puces peuvent transmettre des maladies bactériennes aux chats, dont la maladie des griffes du chat, maladie que les chats peuvent ensuite inoculer à l'Homme. Les puces des chiens et des chats sont aussi les vecteurs d'un ver digestif appelé *Dipylidium caninum.*

Les puces des animaux de compagnie peuvent aussi occasionnellement piquer les humains, provoquant boutons et démangeaisons. Ceci arrive surtout lorsqu'il existe un grand nombre de jeunes adultes dans l'environnement. Appelés improprement « puces de parquet », ou encore « puces de plancher », il s'agit en fait de puces de chiens ou de chats adultes pré-émergeantes, qui éclosent soudainement et en grand nombre lorsqu'elles ressentent les vibrations produites par les pas d'une personne entrant dans une pièce. Ces jeunes puces affamées piquent alors l'humain qui passe à leur portée.

Il existe de nombreux produits pour traiter les animaux de compagnie contre les puces. Ils se différencient par leur composition, leur forme, leur durée d'action.

La plupart sont des médicaments vétérinaires qui nécessitent ou pas une prescription. Molécules chimiques dont la galénique peut présenter un risque toxique pour le jeune enfant. C'est le cas notamment des colliers antiparasitaires et des spot-on couramment proposés par les vétérinaires, qui paradoxalement ne nécessitent plus de prescription et sont disponibles en libres services dans les magasins spécialisés.

D'autres galéniques comme le comprimé présentent moins de risque mais, avec une rémanence généralement d'un mois, ces molécules sollicitent beaucoup le foie de l'animal.

D'autres part, il existe une volonté des propriétaires d'avoir des traitements préventifs simple et qui ne sont pas sans effet secondaire pour les animaux.

La nouvelle réglementation qui concerne les additifs sensoriels à usage vétérinaire permet un retour à l'utilisation d'extrait de plantes aromatiques permettant de palier l'utilisation de compositions chimiques dans certains cas et atteindre les objectifs recherchés. Ceci est le cas dans la dermatite digitée comme le propose le brevet français FR3026643.

Toutefois, il n'existe à ce jour pas de de telle composition contre les puces.

Aussi, l'invention a pour but de pallier ces manques de l'art antérieur.

Un des buts de l'invention est de fournir un moyen naturel et efficace de prévenir et/ou d'éliminer les puces chez les animaux d'élevage, cela sans intervention de produits chimiques.

Un autre but de l'invention est de fournir un procédé de fabrication simple de ce moyen de d'éliminer les puces.

Aussi, l'invention concerne-t-elle une composition consistant essentiellement en au moins un extrait de chacune des plantes aromatiques suivantes :
- les plantes aromatiques de la famille des *Lamiacées,*
- les plantes aromatiques de la famille des *Astéracées,* et
- les plantes aromatiques de la famille des *Poacées,*
- et éventuellement les plantes aromatiques de la famille des *Fabacées,*
ladite composition consistant en un mélange d'un extrait de mélisse, d'un extrait de thym, d'un extrait de romarin d'un extrait d'absinthe et d'un extrait de lemongrass

Les inventeurs on fait la constatation de, de manière surprenante, que les extraits issus des parties aériennes, des racines, des semences ou des fruits secs, de certaines plantes aromatiques décrites ci-dessus ou l'un de leurs mélanges présentent un effet significatif permettant de protéger d'une infestation par les puces.

Les inventeurs ont notamment fait la constatation surprenant que le mélange d'au moins un extrait de *Lamiacées, d'Astéracées* et de *Poacées* susmentionné exerçait cet effet

Les Lamiacées, sont des plantes spermaphytes, angiospermes, dicotylédones et gamopétales dont il existe dans la flore française environ 200 espèces connues. Il s'agit de plantes herbacées, annuelles ou vivaces adaptées à la sécheresse et aux climats de type méditerranéen. Structurellement, ces plantes ont une tige quadrangulaire, leurs feuilles sont simples, opposées et de formes très variables. Certaines feuilles sont enroulées par-dessous, souvent coriaces et épaisses pour mieux résister à la sécheresse.

La famille des Astéracées (anciennement nommées « composées ») est une importante famille de plantes dicotylédones (principalement herbacées) qui comprend près de 13000 espèces réparties en 1500 genres. Ces plantes ont la caractéristique commune d'avoir une inflorescence en capitule, c'est-à-dire une multitude de fleurs sans pédoncule regroupées sur un réceptacle et entourées de bractées florales. On les retrouve arides à semi-arides de latitudes subtropicales et tempérées inférieures.

Les Poacées, ou communément appelées les graminées, sont des angiospermes monocotylédones représentes par plus de 12 000 espèces, à répartition cosmopolite. Ce sont généralement des plantes herbacées, plus rarement ligneuses, qui partagent des caractéristiques morphologiques qui les distinguent nettement des autres familles végétales : des tiges cylindriques aux entrenoeuds creux, des feuilles alternes à disposition distique, une inflorescence élémentaire en épillets, des fleurs réduites aux organes sexuels et des fruits dont le péricarpe est soudé à la graine).

Les Fabacées, ou Légumineuses, forment une famille de plantes angiospermes dicotylédones regroupant plus de 19 000 espèces. Les Fabacées, au sens large, sont des plantes herbacées, des arbustes, des arbres ou des lianes. Leur répartition est cosmopolite, et on, les retrouve sur tous les continents, des zones froides aux zones tropicales.

Dans ce qui précède et ce qui suit, « au moins un extrait » signifie un extrait ou deux extraits, ou trois extraits ou plus d'extraits de chacune des plantes aromatiques suscitées.

Aussi, avantageusement, la composition susmentionnée peut comprendre
- deux extraits de plantes aromatiques de la famille des *Lamiacées,*
- un extrait de plantes aromatiques de la famille des *Astéracées,* et
- un extrait de plantes aromatiques de la famille des *Poacées,*
et éventuellement un extrait plantes aromatiques de la famille des *Fabacées.*

Encore plus avantageusement, la composition susmentionnée peut comprendre
- trois extraits de plantes aromatiques de la famille des *Lamiacées,*
- un extrait de plantes aromatiques de la famille des *Astéracées,* et
- un extrait de plantes aromatiques de la famille des *Poacées,*
et éventuellement un extrait plantes aromatiques de la famille des *Fabacées.*

De manière avantageuse, l'invention concerne la composition susmentionnée, dans laquelle ledit au moins un extrait de Lamiacées représente en masse de 5 à 50%, ledit au moins un extrait d'Astéracées représente en masse de 1 à 20% et ledit au moins un extrait de Poacées représente en masse de 1 à 1 0%, et éventuellement ledit au moins un extrait de Fabacées représente en masse de 5 % à 15 %,
les pourcentages étant exprimés en masse par rapport à la masse totale de la composition.

Il est avantageux que la composition selon l'invention comprenne :
- de 5 à 50% en masse d'au moins un extrait de Lamiacées, notamment de 20 % à 40 % en masse d'au moins un extrait de Lamiacées,
- de 1 à 20% en masse d'au moins un extrait d'Astéracées, notamment de 5 % à 15 % % en masse d'au moins un extrait d'Astéracées, et
- de 1 % à 10 % en masse d'au moins un extrait de Poacées, notamment de 1 % à 5 % en masse d'au moins un extrait Poacées,
- et éventuellement de 5 % à 15 % en masse d'au moins un extrait de Fabacées, notamment de 8 % à 12 % en masse d'au moins un extrait de Fabacées.

De manière avantageuse, la composition comprend :
- de 5 à 50% en masse de deux ou de trois extraits de Lamiacées, notamment de 20 % à 40 % en masse de deux ou de trois extraits de Lamiacées,
- de 1 à 20% en masse d'au moins un extrait d'Astéracées, notamment de 5 % à 15 % % en masse d'au moins un extrait d'Astéracées, et
- de 1 % à 10 % en masse d'au moins un extrait de Poacées, notamment de 1 % à 5 % en masse d'au moins un extrait Poacées,
- et éventuellement de 5 % à 15 % en masse d'au moins un extrait de Fabacées, notamment de 8 % à 12 % en masse d'au moins un extrait de Fabacées.

Avantageusement, l'invention concerne la composition susmentionnée, comprenant, ou consistant essentiellement en, ou consistant en un mélange d'un extrait de mélisse, d'un extrait de thym, d'un extrait de romarin, d'un extrait d'absinthe et d'un extrait de lemongrass, et éventuellement un extrait de fenugrec.

Le Thym convenant pour la présente invention, notamment *Thymus V,* est une plante originaire du bassin méditerranéen se présentant sous forme d'un sousarbrisseau. Cette plante est généralement utilisée pour des applications : alimentaires (salade, boisson énergisante) aussi bien chez l'homme que chez l'animal, agricoles comme plante compagnonne pour protéger les cultures des insectes.

Le Romarin convenant pour la présente invention, notamment *Rosmarinus O,* est une plante originaire du bassin méditerranéen se présentant sous forme d'un arbrisseau. Cette plante est généralement utilisée pour des applications : alimentaires (salade, boisson énergisante) aussi bien chez l'homme que chez l'animal, agricoles comme plante compagnonne pour protéger les cultures des insectes.

La Mélisse convenant pour la présente invention, notamment *Melissa O,* est une plante se présentant sous forme d'une plante vivace. Cette plante est généralement utilisée pour des applications : alimentaires (boisson, bonbon) aussi bien chez l'homme que chez l'animal, agricoles comme plante compagnonne pour protéger les cultures des insectes.

Le Fenugrec, notamment *Trigonella Fornum G,* est une plante herbacée caractérisée par des feuilles trifoliolées et par un fruit sous forme de gousse contenant les graines. Cette plante est généralement utilisée pour des applications : alimentaires (boisson, bonbon) aussi bien chez l'homme que chez l'animal, agricoles comme plante compagnonne pour protéger les cultures des insectes.

L'absinthe, notamment *Artemisia absinthium L.,* est une espèce de plantes de la famille des Astéracées. Plante vivace, herbacée recouverte de poils soyeux blancs argentés et de nombreuses glandes oléifères. La tige est de couleur vert argent, droite, cannelée, ramifiée et très feuillée. Cette plante est généralement utilisée pour des applications : alimentaires (boisson, bonbon) aussi bien chez l'homme que chez l'animal, agricoles comme plante compagnonne pour protéger les cultures des insectes.

Herbacée et aromatique, le Lemongrass, notamment *Cymbopogon citratus,* est une plante vivace rhizomateuse. C'est une excellente compagne au potager car elle éloigne les mineuses. Cette plante est généralement utilisée pour des applications : alimentaires (boisson, bonbon) aussi bien chez l'homme que chez l'animal, agricoles comme plante compagnonne pour protéger les cultures des insectes.

De manière plus avantageuse, l'invention concerne la composition susmentionnée, comprenant, ou consistant essentiellement en, ou consistant en :
- de 5 % à 50 % d'extrait de mélisse,
- de 10 % à 50 % d'extrait de thym,
- de 10 % à 50 % d'extrait de romarin,
- de 1 % à 20% d'extrait d'absinthe, et
- de 1 % à 10 % d'extrait de lemongrass,
- et éventuellement de 5 % à 15 % d'extrait de fenugrec,
les pourcentages étant exprimés en masse par rapport à la masse totale de la composition.

De manière plus avantageuse, l'invention concerne la composition susmentionnée, comprenant, ou consistant essentiellement en, ou consistant en :
- de 15 % à 40 % d'extrait de mélisse,
- de 20 % à 40 % d'extrait de thym,
- de 20 % à 40 % d'extrait de romarin,
- de 5 % à 15 % d'extrait d'absinthe, et
- de 1 % à 5 % d'extrait de lemongrass,
- et éventuellement de 8 % à 12 % d'extrait de fenugrec,
les pourcentages étant exprimés en masse par rapport à la masse totale de la composition.

D'autres compositions avantageuses selon l'invention sont les compositions suivantes :
Composition A :

| | |
|---|---|
| Extrait de Thym : | 40% en masse |
| Extrait de Romarin | 25% en masse |
| Extrait de Mélisse | 15% en masse |
| Extrait de Fenugrec | 1 0% en masse |
| Extrait d'Absinthe | 5% en masse |
| Extrait de Lemongrass | 5% en masse |

les pourcentages étant exprimés en masse par rapport à la masse totale de la composition.
Composition B :

| | |
|---|---|
| Extrait de Thym : | 20% en masse |
| Extrait de Romarin | 40% en masse |
| Extrait de Mélisse | 20% en masse |
| Extrait de Fenugrec | 1 0% en masse |
| Extrait d'Absinthe | 5% en masse |
| Extrait de Lemongrass | 5% en masse |

les pourcentages étant exprimés en masse par rapport à la masse totale de la composition.
Composition C :

| | |
|---|---|
| Extrait de Thym : | 25% en masse |
| Extrait de Romarin | 20% en masse |
| Extrait de Mélisse | 37% en masse |
| Extrait d'Absinthe | 15% en masse |
| Extrait de Lemongrass | 1 % en masse |

les pourcentages étant exprimés en masse par rapport à la masse totale de la composition.

Avantageusement, la composition selon l'invention peut également contenir des vitamines, ou des acides aminés ou des minéraux, ou un mélange de ceux-ci

L'invention concerne également une composition à usage vétérinaire ou à usage pharmaceutique comprenant, ou consistant essentiellement en, ou consistant en à titre de substance active la composition telle que définie ci-dessus, en association avec un véhicule acceptable, notamment acceptable pharmaceutiquement ou physiologiquement acceptable.

La composition selon l'invention pouvant être utilisée en alimentation, vétérinaire ou pharmaceutique est prévue avantageusement pour administration orale, et peut faire l'objet d'une formulation classique en utilisant un ou plusieurs véhicules ou excipients physiologiquement acceptables.

Pour administration orale, les compositions pharmaceutiques ou vétérinaires peuvent être par exemple sous la forme de comprimés, comprenant des comprimés à mâcher ou à sucer, ou de capsules de gélatine dures ou molles, y compris des capsules de gélatine molles à mâcher. Ces compositions peuvent être préparées par des méthodes classiques avec des excipients pharmaceutiquement acceptables tels que des liants (par exemple de l'amidon de mais prégélifié, de la polyvinylpyrrolidone ou de l'hydroxypropyl méthyl- cellulose); des charges (par exemple du lactose, de la cellulose microcristalline ou de l'hydrogénophosphate de calcium); des lubrifiants (par exemple du stéarate de magnésium, du talc ou de la silice); des agents de -5- désagrégation (par exemple de l'amidon de pomme de terre ou du glycolate d'amidon sodique); ou des agents mouillants (par exemple du laurylsulfate de sodium).

Les comprimés peuvent être enrobés par des méthodes bien connues dans l'état de la technique. Des préparations liquides pour administration orale peuvent être sous la forme, par exemple, de solutions, sirops ou suspensions, ou elles peuvent être présentées sous la forme d'un produit sec (sous la forme, par exemple, d'une présentation en sachets) pour constitution avec de l'eau ou un autre véhicule approprié avant emploi. De telles compositions liquides peuvent être préparées par des méthodes classiques avec des additifs pharmaceutiquement acceptables tels que des agents de suspension (par exemple du sirop de sorbitol, des dérivés de cellulose ou des graisses comestibles hydrogénées); des agents émulsionnants (par exemple de la lécithine ou de la gomme arabique); des véhicules non-aqueux (par exemple de l'huile d'amande, des esters huileux, de l'alcool éthylique ou des huiles végétales fractionnées); et des conservateurs (par exemple des 2-hydroxybenzoates de méthyle ou de propyle ou de l'acide sorbique).

Les préparations peuvent contenir aussi des sels jouant le rôle de tampon, des agents aromatisants, colorants et/ou édulcorants, selon ce qui est approprié.

Des agents aromatisants utilisables comprennent par exemple des arômes de fruits, de la menthe poivrée ou de la réglisse ou des agents appétant. Les agents édulcorants peuvent être par exemple des édulcorants naturels tels que des sucres (par exemple le sucrose ou le fructose) et/ou des édulcorants intenses (par exemple l'aspartame, stevia etc...).

L'invention concerne également un aliment destiné à l'alimentation des animaux non humain comprenant de 0,01 % à 100 % d'une composition définie ci-dessus, les pourcentages étant exprimés en masse par rapport à la masse totale de l'aliment.

L'aliment selon l'invention est soit une boisson, soit un aliment solide.

Dans le cadre de l'aliment susmentionné, la composition selon l'invention est avantageusement sous forme d'une solution, d'une suspension aqueuse à apporter dans l'eau de boisson ou un aliment liquide, ou à l'état sec, adsorbé sur support pulvérulent ainsi que toute autre forme d'apport à l'alimentation animale.

L'aliment peut être constitué de la seule composition telle que définie ci-dessus, ou être constitué d'un aliment couramment utilisé en nutrition animale bien connu de l'art antérieur (apport de protéine, de lipides, glucides, minéraux et vitamines...) auquel on ajoute de 0,01% à 99,99% de la composition susmentionnée.

Dans l'invention, de 0,01% à 100% correspond à 0,01%, 0,02%, 0,03%, 0,04%, 0,05%, 0,06%, 0,07%, 0,08%, 0,09%, 0,1%, 0,2%, 0,3%, 0,4%, 0,5%, 0,6%, 0,7%, 0,8%, 0,9%, 1 %, 1,1%, 1,2%, 1,3%, 1,4%, 1,5%, 1,6%, 1,7%, 1,8%, 1,9%, 2%, 2,1%, 2,2%, 2,3%, 2,4%, 2,5%, 2,6%, 2,7%, 2,8%, 2,9%, 3%, 3,1%, 3,2%, 3,3%, 3,4%, 3,5%, 3,6%, 3,7%, 3,8%, 3,9%, 4%, 4,1%, 4,2%, 4,3%, 4,4%, 4,5%, 4,6%, 4,7%, 4,8%, 4,9%, 5%, 5,1%, 5,2%, 5,3%, 5,4%, 5,5%, 5,6%, 5,7%, 5,8%, 5,9%, 6%, 6,1%, 6,2%, 6,3%, 6,4%, 6,5%, 6,6%, 6,7%, 6,8%, 6,9%, 7%, 7,1%, 7,2%, 7,3%, 7,4%, 7,5%, 7,6%, 7,7%, 7,8%, 7,9%, 8%, 8,1%, 8,2%, 8,3%, 8,4%, 8,5%, 8,6%, 8,7%, 8,8%, 8,9%, 9%, 9,1%, 9,2%, 9,3%, 9,4%, 9,5%, 9,6%, 9,7%, 9,8%, 9,9%, 10%, 10,1%, 10,2%, 10,3%, 10,4%, 10,5%, 10,6%, 10,7%, 10,8%, 10,9%, 11%, 11,1%, 11,2%, 11,3%, 11,4%, 11,5%, 11,6%, 11,7%, 11,8%, 11,9%, 12%, 12,1%, 12,2%, 12,3%, 12,4%, 12,5%, 12,6%, 12,7%, 12,8%, 12,9%, 13%, 13,1%, 13,2%, 13,3%, 13,4%, 13,5%, 13,6%, 13,7%, 13,8%, 13,9%, 14%, 14,1%, 14,2%, 14,3%, 14,4%, 14,5%, 14,6%, 14,7%, 14,8%, 14,9%, 15%, 15,1%, 15,2%, 15,3%, 15,4%, 15,5%, 15,6%, 15,7%, 15,8%, 15,9%, 16%, 16,1%, 16,2%, 16,3%, 16,4%, 16,5%, 16,6%, 16,7%, 16,8%, 16,9%, 17%, 17,1%, 17,2%, 17,3%, 17,4%, 17,5%, 17,6%, 17,7%, 17,8%, 17,9%, 18%, 18,1%, 18,2%, 18,3%, 18,4%, 18,5%, 18,6%, 18,7%, 18,8%, 18,9%, 19%, 19,1%, 19,2%, 19,3%, 19,4%, 19,5%, 19,6%, 19,7%, 19,8%, 19,9%, 20%, 20,1%, 20,2%, 20,3%, 20,4%, 20,5%, 20,6%, 20,7%, 20,8%, 20,9%, 21%, 21,1%, 21,2%, 21,3%, 21,4%, 21,5%, 21,6%, 21,7%, 21,8%, 21,9%, 22%, 22,1%, 22,2%, 22,3%, 22,4%, 22,5%, 22,6%, 22,7%, 22,8%, 22,9%, 23%, 23,1%, 23,2%, 23,3%, 23,4%, 23,5%, 23,6%, 23,7%, 23,8%, 23,9%, 24%, 24,1%, 24,2%, 24,3%, 24,4%, 24,5%, 24,6%, 24,7%, 24,8%, 24,9%, 25%, 25,1%, 25,2%, 25,3%, 25,4%, 25,5%, 25,6%, 25,7%, 25,8%, 25,9%, 26%, 26,1%, 26,2%, 26,3%, 26,4%, 26,5%, 26,6%, 26,7%, 26,8%, 26,9%, 27%, 27,1%, 27,2%, 27,3%, 27,4%, 27,5%, 27,6%, 27,7%, 27,8%, 27,9%, 28%, 28,1%, 28,2%, 28,3%, 28,4%, 28,5%, 28,6%, 28,7%, 28,8%, 28,9%, 29%, 29,1%, 29,2%, 29,3%, 29,4%, 29,5%, 29,6%, 29,7%, 29,8%, 29,9%, 30%, 30,1%, 30,2%, 30,3%, 30,4%, 30,5%, 30,6%, 30,7%, 30,8%, 30,9%, 31%, 31,1%, 31,2%, 31,3%, 31,4%, 31,5%, 31,6%, 31,7%, 31,8%, 31,9%, 32%, 32,1%, 32,2%, 32,3%, 32,4%, 32,5%, 32,6%, 32,7%, 32,8%, 32,9%, 33%, 33,1%, 33,2%, 33,3%, 33,4%, 33,5%, 33,6%, 33,7%, 33,8%, 33,9%, 34%, 34,1%, 34,2%, 34,3%, 34,4%, 34,5%, 34,6%, 34,7%, 34,8%, 34,9%, 35%, 35,1%, 35,2%, 35,3%, 35,4%, 35,5%, 35,6%, 35,7%, 35,8%, 35,9%, 36%, 36,1%, 36,2%, 36,3%, 36,4%, 36,5%, 36,6%, 36,7%, 36,8%, 36,9%, 37%, 37,1%, 37,2%, 37,3%, 37,4%, 37,5%, 37,6%, 37,7%, 37,8%, 37,9%, 38%, 38,1%, 38,2%, 38,3%, 38,4%, 38,5%, 38,6%, 38,7%, 38,8%, 38,9%, 39%, 39,1%, 39,2%, 39,3%, 39,4%, 39,5%, 39,6%, 39,7%, 39,8%, 39,9%, 40%, 40,1%, 40,2%, 40,3%, 40,4%, 40,5%, 40,6%, 40,7%, 40,8%, 40,9%, 41%, 41,1%, 41,2%, 41,3%, 41,4%, 41,5%, 41,6%, 41,7%, 41,8%, 41,9%, 42%, 42,1%, 42,2%, 42,3%, 42,4%, 42,5%, 42,6%, 42,7%, 42,8%, 42,9%, 43%, 43,1%, 43,2%, 43,3%, 43,4%, 43,5%, 43,6%, 43,7%, 43,8%, 43,9%, 44%, 44,1%, 44,2%, 44,3%, 44,4%, 44,5%, 44,6%, 44,7%, 44,8%, 44,9%, 45%, 45,1%, 45,2%, 45,3%, 45,4%, 45,5%, 45,6%, 45,7%, 45,8%, 45,9%, 46%, 46,1%, 46,2%, 46,3%, 46,4%, 46,5%, 46,6%, 46,7%, 46,8%, 46,9%, 47%, 47,1%, 47,2%, 47,3%, 47,4%, 47,5%, 47,6%, 47,7%, 47,8%, 47,9%, 48%, 48,1%, 48,2%, 48,3%, 48,4%, 48,5%, 48,6%, 48,7%, 48,8%, 48,9%, 49%, 49,1%, 49,2%, 49,3%, 49,4%, 49,5%, 49,6%, 49,7%, 49,8%, 49,9%, 50%, 50,1%, 50,2%, 50,3%, 50,4%, 50,5%, 50,6%, 50,7%, 50,8%, 50,9%, 51%, 51,1%, 51,2%, 51,3%, 51,4%, 51,5%, 51,6%, 51,7%, 51,8%, 51,9%, 52%, 52,1%, 52,2%, 52,3%, 52,4%, 52,5%, 52,6%, 52,7%, 52,8%, 52,9%, 53%, 53,1%, 53,2%, 53,3%, 53,4%, 53,5%, 53,6%, 53,7%, 53,8%, 53,9%, 54%, 54,1%, 54,2%, 54,3%, 54,4%, 54,5%, 54,6%, 54,7%, 54,8%, 54,9%, 55%, 55,1%, 55,2%, 55,3%, 55,4%, 55,5%, 55,6%, 55,7%, 55,8%, 55,9%, 56%, 56,1%, 56,2%, 56,3%, 56,4%, 56,5%, 56,6%, 56,7%, 56,8%, 56,9%, 57%, 57,1%, 57,2%, 57,3%, 57,4%, 57,5%, 57,6%, 57,7%, 57,8%, 57,9%, 58%, 58,1%, 58,2%, 58,3%, 58,4%, 58,5%, 58,6%, 58,7%, 58,8%, 58,9%, 59%, 59,1%, 59,2%, 59,3%, 59,4%, 59,5%, 59,6%, 59,7%, 59,8%, 59,9%, 60%, 60,1%, 60,2%, 60,3%, 60,4%, 60,5%, 60,6%, 60,7%, 60,8%, 60,9%, 61%, 61,1%, 61,2%, 61,3%, 61,4%, 61,5%, 61,6%, 61,7%, 61,8%, 61,9%, 62%, 62,1%, 62,2%, 62,3%, 62,4%, 62,5%, 62,6%, 62,7%, 62,8%, 62,9%, 63%, 63,1%, 63,2%, 63,3%, 63,4%, 63,5%, 63,6%, 63,7%, 63,8%, 63,9%, 64%, 64,1%, 64,2%, 64,3%, 64,4%, 64,5%, 64,6%, 64,7%, 64,8%, 64,9%, 65%, 65,1%, 65,2%, 65,3%, 65,4%, 65,5%, 65,6%, 65,7%, 65,8%, 65,9%, 66%, 66,1%, 66,2%, 66,3%, 66,4%, 66,5%, 66,6%, 66,7%, 66,8%, 66,9%, 67%, 67,1%, 67,2%, 67,3%, 67,4%, 67,5%, 67,6%, 67,7%, 67,8%, 67,9%, 68%, 68,1%, 68,2%, 68,3%, 68,4%, 68,5%, 68,6%, 68,7%, 68,8%, 68,9%, 69%, 69,1%, 69,2%, 69,3%, 69,4%, 69,5%, 69,6%, 69,7%, 69,8%, 69,9%, 70%, 70,1%, 70,2%, 70,3%, 70,4%, 70,5%, 70,6%, 70,7%, 70,8%, 70,9%, 71%, 71,1%, 71,2%, 71,3%, 71,4%, 71,5%, 71,6%, 71,7%, 71,8%, 71,9%, 72%, 72,1%, 72,2%, 72,3%, 72,4%, 72,5%, 72,6%, 72,7%, 72,8%, 72,9%, 73%, 73,1%, 73,2%, 73,3%, 73,4%, 73,5%, 73,6%, 73,7%, 73,8%, 73,9%, 74%, 74,1%, 74,2%, 74,3%, 74,4%, 74,5%, 74,6%, 74,7%, 74,8%, 74,9%, 75%, 75,1%, 75,2%, 75,3%, 75,4%, 75,5%, 75,6%, 75,7%, 75,8%, 75,9%, 76%, 76,1%, 76,2%, 76,3%, 76,4%, 76,5%, 76,6%, 76,7%, 76,8%, 76,9%, 77%, 77,1%, 77,2%, 77,3%, 77,4%, 77,5%, 77,6%, 77,7%, 77,8%, 77,9%, 78%, 78,1%, 78,2%, 78,3%, 78,4%, 78,5%, 78,6%, 78,7%, 78,8%, 78,9%, 79%, 79,1%, 79,2%, 79,3%, 79,4%, 79,5%, 79,6%, 79,7%, 79,8%, 79,9%, 80%, 80,1%, 80,2%, 80,3%, 80,4%, 80,5%, 80,6%, 80,7%, 80,8%, 80,9%, 81%, 81,1%, 81,2%, 81,3%, 81,4%, 81,5%, 81,6%, 81,7%, 81,8%, 81,9%, 82%, 82,1%, 82,2%, 82,3%, 82,4%, 82,5%, 82,6%, 82,7%, 82,8%, 82,9%, 83%, 83,1%, 83,2%, 83,3%, 83,4%, 83,5%, 83,6%, 83,7%, 83,8%, 83,9%, 84%, 84,1%, 84,2%, 84,3%, 84,4%, 84,5%, 84,6%, 84,7%, 84,8%, 84,9%, 85%, 85,1%, 85,2%, 85,3%, 85,4%, 85,5%, 85,6%, 85,7%, 85,8%, 85,9%, 86%, 86,1%, 86,2%, 86,3%, 86,4%, 86,5%, 86,6%, 86,7%, 86,8%, 86,9%, 87%, 87,1%, 87,2%, 87,3%, 87,4%, 87,5%, 87,6%, 87,7%, 87,8%, 87,9%, 88%, 88,1%, 88,2%, 88,3%, 88,4%, 88,5%, 88,6%, 88,7%, 88,8%, 88,9%, 89%, 89,1%, 89,2%, 89,3%, 89,4%, 89,5%, 89,6%, 89,7%, 89,8%, 89,9%, 90%, 90,1%, 90,2%, 90,3%, 90,4%, 90,5%, 90,6%, 90,7%, 90,8%, 90,9%, 91%, 91,1%, 91,2%, 91,3%, 91,4%, 91,5%, 91,6%, 91,7%, 91,8%, 91,9%, 92%, 92,1%, 92,2%, 92,3%, 92,4%, 92,5%, 92,6%, 92,7%, 92,8%, 92,9%, 93%, 93,1%, 93,2%, 93,3%, 93,4%, 93,5%, 93,6%, 93,7%, 93,8%, 93,9%, 94%, 94,1%, 94,2%, 94,3%, 94,4%, 94,5%, 94,6%, 94,7%, 94,8%, 94,9%, 95%, 95,1%, 95,2%, 95,3%, 95,4%, 95,5%, 95,6%, 95,7%, 95,8%, 95,9%, 96%, 96,1%, 96,2%, 96,3%, 96,4%, 96,5%, 96,6%, 96,7%, 96,8%, 96,9%, 97%, 97,1%, 97,2%, 97,3%, 97,4%, 97,5%, 97,6%, 97,7%, 97,8%, 97,9%, 98%, 98,1%, 98,2%, 98,3%, 98,4%, 98,5%, 98,6%, 98,7%, 98,8%, 98,9%, 99%, 99,1%, 99,2%, 99,3%, 99,4%, 99,5%, 99,6%, 99,7%, 99,8%, 99,9%, 99,91%, 99,92%, 99,93%, 99,94%, 99,95%, 99,96%, 99,97%, 99,98%, 99,99% ou 100%.

L'invention concerne en outre la composition susmentionnée en tant que médicament, ou pour son utilisation en tant que médicament à usage notamment vétérinaire.

L'invention concerne en outre la composition susmentionnée en tant qu'aliment complémentaire, agent de saveur ou autre produit destiné à l'alimentation.

Dans le cadre d'un médicament à usage vétérinaire, il est avantageux de disposer d'une composition sous forme liquide à utiliser pure ou à ajouter dans une eau de boisson (pour dilution), ou à l'état sec adsorbé sur support alimentaire, notamment un support pulvérulent.

L'aliment complémentaire est avantageusement administré, ou sous une forme apte à être administrée, durant toute la vie de l'animal, de l'ordre de 1 mL à 20 mL par animal, notamment une fois par jour.

L'invention concerne également la composition susmentionnée pour son utilisation dans le cadre du traitement des mammifères domestiques pour la prévention ou le traitement contre l'infestation par des puces (aussi appelée pulicose), et de la prévention des symptômes associés.

Les mammifères domestiques selon l'invention sont les mammifères de compagnie pouvant être infectés par des puces, et notamment sans être limitatif : les chats et les chiens, les hérissons, les lapins. La composition selon l'invention est notamment avantageuse pour les chiens et les chats.

Comme il est montré dans les exemples ci-dessous, la composition selon l'invention permet de limiter l'infestation par des puces, et donc de prévenir une infestation. Elle permet également de prévenir les complications et symptômes liées à la présence de puces, telles que le prurit, les érythèmes, les squames, les lésions secondaires (excoriations, croûtes...), la dermatite allergique, et les infections par des microorganismes telles que la lichénification ou encore les infections par des bactéries responsable de la maladie des griffes du chat, ou les infections par les vers digestifs *Dipylidium caninum,* ou le ténia.

L'invention concerne par ailleurs un procédé de préparation de la composition susmentionnée, dans lequel :
- on tasse des feuilles mélisse, de thym, de romarin, d'absinthe et de lemongrass, dans un volume d'eau suffisant pour les recouvrir, pour obtenir un mélange de feuilles tassées,
- on laisse macérer pendant au moins deux semaines à température ambiantes ledit mélange de feuilles tassées, en mélangeant régulièrement ledit mélange de feuilles tassées,
- on élimine le macérat pour conserver la fraction liquide,
- et éventuellement, on place sous agitation mécanique la fraction liquide pour obtenir une composition homogène.

La composition selon l'invention est donc obtenue par un procédé simple peu coûteux, et ne faisant intervenir aucun agent chimique.

La composition est obtenue sous une forme liquide à l'issue du procédé susmentionné, et comme mentionnée ci-dessus, peut être utilisée directement sous forme liquide pure ou additionnée à une eau de boisson, sous mélangée à un aliment, par exemple des croquettes, soit par imprégnation des croquettes, soit par adjonction de la composition adsorbée sur un support pulvérulent.

L'invention sera mieux comprise à la lumière de la figure et des exemples suivants :

### Brève description de la figure

[Fig. 1]

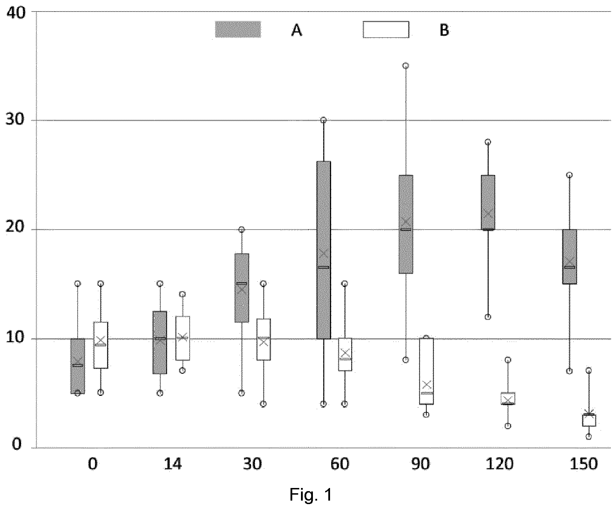

La **Figure 1** est un diagramme en boite (boites à moustaches) représentant la comparaison des distributions du nombre de puces par animal (axe y) en fonction de l'aliment reçu (lot A en blanc ou lot B en gris) et en fonction du temps (axe x ; 0 = début de l'étude). La ligne horizontale dans la boite indique la médiane, les limites de la boite indiquent les 25^{èmes} et 75^{èmes} percentiles et les moustaches indiquent les valeurs des résultats maximales et minimales. La croix (x) à l'intérieur de la boite représente la moyenne des données.

### Exemples

### Exemple 1 : Procédé de préparation d'une composition selon l'invention

Le procédé de fabrication de l'aliment complémentaire comprend les étapes consistant à :
- Introduire dans une cuve de l'eau. Le volume du mélange correspond au tiers du volume de la cuve.
- Introduire les feuilles des plantes aromatiques en commençant par la quantité la plus faible.
- Tasser les plantes au moyen d'une spatule.
- Rajouter, si nécessaire, un mélange eau pour recouvrir complètement les plantes.
- Couvrir la cuve avec le capot et laisser macérer à température ambiante (de 15 à 27°C) pendant 2 semaines minimum, tout en agitant régulièrement le mélange au moyen d'une spatule, notamment tous les 3 jours.
- Filtrer le mélange par filtration clarifiante sur tamis.
- Ajouter éventuellement les actifs supplémentaires (minéraux, vitamines, extraits...).
- Agiter l'extrait au moyen d'un agitateur mécanique réglé à environs 100 rpm pendant 12 heures pour obtenir une composition homogène.

La composition ainsi obtenue est éventuellement pulvérisée dans un mélangeur contenant un support alimentaire pulvérulent (avant d'être conditionné, pour obtenir un aliment comprenant la composition selon l'invention.

L'aliment est ainsi prêt à être distribué aux animaux.

Enfin, les constituants de la composition peuvent être stérilisés, notamment par chauffage et/ou par passage sur une membrane. Ils peuvent être désinfectés, également par chauffage ou encore par l'intermédiaire d'un agent désinfectant.

### Exemple 2 : Mesure de l'efficacité de la composition selon l'invention

### 1- But de l'étude.

Le but de cette étude était d'évaluer, dans un cadre double aveugle contrôlé contre placebo, l'efficacité de la composition selon l'invention utilisée seule, en période estivale, pour limiter les populations de puces chez des chiens infestés naturellement et vivant dans des chenils en terre battue sans traitement de l'environnement.

### 2- Matériel et méthode.

### 2.1 les animaux.

Vingt-deux chiens adultes âgés de 1 à 11 ans, en bonne santé, hébergés dans 7 chenils semi-ouverts, par groupes de 1 à 5 ont été inclus. Leur nourriture était exclusivement composée de l'aliment fourni par le promoteur de l'étude, distinguable uniquement par la lettre figurant sur l'emballage extérieur (A ou B). De l'eau était à disposition à volonté. Pendant toute la durée de l'étude, les conditions d'hébergement et de nourriture sont restées stables. Aucun médicament antiparasitaire n'était autorisé. Aucun traitement antiparasitaire externe n'avait été administré au cours de 6 derniers mois. Le signalement de chaque animal figure dans la **table 1.**

### [Table 1]

**Table 1 : signalement des animaux**

| **N° dans l'étude** | **Sexe** | **Age (ans)** | **Race** | **Chenil** | **Poids (kg)** | **Groupe** |
|---|---|---|---|---|---|---|
| 1 | M | 8 | Bruno du Jura | 1 | 27 | A |
| 2 | M | 3 | Griffon | 1 | 24 | A |
| 3 | F | 11 | Bruno du Jura | 1 | 25 | A |
| 4 | F | 11 | Bruno du Jura | 1 | 27 | A |
| 10 | F | 6 | Bleu de Gascogne | 3 | 24 | A |
| 11 | M | 1 | Griffon | 3 | 18 | A |
| 12 | F | 2 | Bleu de Gascogne | 3 | 20 | A |
| 13 | F | 3 | Griffon | 3 | 19 | A |
| 19 | M | 3 | Griffon | 6 | 25 | A |
| 20 | M | 7 | Griffon | 6 | 35 | A |
| 21 | M | 3 | Griffon | 6 | 23 | A |
| 22 | M | 7 | Griffon | 7 | 30 | A |
| 5 | M | 6 | Bruno du Jura | 2 | 27 | B |
| 6 | M | 5 | Bleu de Gascogne | 2 | 25 | B |
| 7 | M | 1 | Griffon | 2 | 20 | B |
| 8 | M | 3 | Griffon | 2 | 31 | B |
| 9 | M | 1 | Griffon | 2 | 22 | B |
| 14 | M | 5 | Griffon | 4 | 23 | B |
| 15 | M | 7 | Griffon | 4 | 30 | B |
| 16 | M | 1 | Bleu de Gascogne | 4 | 22 | B |
| 17 | M | 2 | Bleu de Gascogne | 4 | 22 | B |
| 18 | M | 5 | Griffon | 5 | 31 | B |

### 2.2 Le produit.

L'actif à évaluer a été incorporé à l'aliment avant envoi au détenteur des animaux. Les sacs (12 kg chacun) étaient uniquement différentiables par la lettre « A » ou « B » figurant sur l'emballage. Les sacs étaient stockés dans un endroit approprié, au sec et à température inférieure à 25°C.

L'aliment est constitué de viandes de poulet déshydratées (43%), de pomme de terre entière déshydratée, de pulpe de pomme de terre, de pois, de graisse de volaille, de crevettes déshydratées (4%), d'hydrolysat de protéines animales, de kaolin (2%), de substances minérales, de farine au lin sans gluten, d'extrait de *Yucca schidigera* déshydraté, de levure de bière déshydratée, de pulpe de chicorée déshydratée, et d'un prémélange d'additifs substances aromatiques aux actifs issus d'agrumes. Cet aliment est constitué en masse par rapport à la masse totale de l'aliment : 34% de protéines brutes, 12% de matières grasses brutes, 3% de cellulose brute, 6% de matière minérale, 0,75% de calcium, 0,47% de phosphore, 0,20% d'oméga 3 et 1,10% d'omega 6, avec un ratio Ca/P de 1,55.

La distribution de l'aliment a été effectuée une fois par jour par le propriétaire, la quantité distribuée a été basée sur les recommandations du fabricant pour des animaux avec une activité intense supérieure à deux heures d'exercices de manière à couvrir correctement les besoins. Au sein d'un même parc, l'ensemble des chiens a reçu le même aliment.

### 2.3 Méthode

### 2.3.1 Evaluation de l'activité antiparasitaire.

Les comptages ont été réalisés selon la méthode recommandée par l'Agence européenne du médicament (EMEA) pour les carnivores lors d'essais terrain (*Guideline for the testing and évaluation of the efficacy of antiparasitic substances for the treatment and prévention of tick and flea infestation in dogs and cats EMEA*/*CVMP*/*EWP*/*005*/*2000 -Rev.3* - *14 juillet 2016*). La population de puces de chaque chien est évaluée par comptage visuel. Des comptages par zone sont effectués sur cinq régions corporelles de chaque animal : ligne dorsale, base de la queue, flanc droit, flanc gauche et région inguinale. Pour chaque zone, le temps de dénombrement est limité à une minute et le comptage est réalisé en écartant les poils à l'aide des deux mains jusqu'à ce que la surface de la zone soit couverte. Pour chaque zone, le nombre maximal de puces compté est limité à 50, ce qui porte à 250 le nombre maximal de puces évalué par animal.

Les comptages ont été effectués avant le début de l'étude (J0), puis 14 jours, 1, 2, 3, 4 et 5 mois après le début de la supplémentation.

### 2.3.2 Evaluation clinique

### 2.3.2.1 Tolérance.

La tolérance immédiate a été évaluée au moment de chaque repas par le propriétaire. Lors de chaque visite de suivi, l'examen clinique local et général a permis d'évaluer la tolérance à moyen terme.

### 2.3.2.2 Suivi dermatologique

L'évaluation a eu lieu avant le début de l'étude (J0), puis 14 jours, 1, 2, 3, 4 et 5 mois après le début de la supplémentation. Pour chaque animal, l'intensité des lésions suivantes a été gradée sur une échelle de 0 à 3 [0 : absence; 1 : présence modérée; 2 : présence moyenne; 3 : présence importante] :
- surface atteinte
- Intensité du prurit
- érythème
- squames
- lésions secondaires (excoriations, croûtes...)
- lichénification

La note clinique correspond à la somme des évaluations par lésion.

### 2.4 Analyse des résultats

Les comptages de puces des deux groupes ont été comparés par un test t pour deux échantillons indépendants avec un seuil de significativité de 5%. Les calculs ont été faits à l'aide du logiciel XLSTAT (Addinsoft, v. 19.02 43358).

### 3 Résultats

### 3.1 Tolérance

Aucune réaction d'intolérance n'a été détectée après la distribution de l'aliment. Lors des examens cliniques postérieurs à l'application, aucune anomalie clinique n'a été détectée.

Le chien 6 a été exclu de l'étude à compter de J60, ayant été déplacé hors de l'élevage.

### 3.2 Efficacité antiparasitaire (table 2 et Figure 1).

A l'inclusion dans l'étude, les chiens hébergeaient entre 5 et 15 puces, soit en moyenne 7.9±3.3 puces pour le lot A (12 chiens) et 9.5 ± 3.6 puces pour le lot B (10 chiens).

Les résultats de l'étude sont illustrés à la **figure 1****,** et dans la **table 2** suivante.

**[Table 2]**

| Lot | Chien | 30-juil J0 | 14-août J14 | 28-août J30 | 25-sept J60 | 26-oct J90 | 20-nov J120 | 18-déc J150 |
|---|---|---|---|---|---|---|---|---|
| A | 1 | 5 | 5 | 20 | 25 | 25 | 25 | 20 |
| | 2 | 8 | 12 | 20 | 30 | 35 | 28 | 25 |
| | 3 | 5 | 7 | 10 | 12 | 25 | 20 | 15 |
| | 4 | 7 | 10 | 10 | 10 | 13 | 15 | 12 |
| | 10 | 5 | 7 | 15 | 10 | 19 | 20 | 15 |
| | 11 | 10 | 14 | 20 | 30 | 12 | 25 | 20 |
| | 12 | 15 | 14 | 15 | 15 | 17 | 20 | 15 |
| | 13 | 10 | 12 | 12 | 10 | 20 | 20 | 16 |
| | 19 | 5 | 6 | 5 | 4 | 8 | 12 | 7 |
| | 20 | 5 | 6 | 15 | 20 | 30 | 28 | 25 |
| | 21 | 12 | 15 | 17 | 18 | 25 | 20 | 17 |
| | 22 | 8 | 10 | 15 | 30 | 20 | 25 | 18 |
| B | 5 | 10 | 12 | 12 | 15 | 4 | 4 | 4 |
| | 6 | 5 | 7 | 8 | - | - | - | - |
| | 7 | 5 | 8 | 4 | 8 | 10 | 5 | 3 |
| | 8 | 8 | 7 | 15 | 6 | 10 | 5 | 3 |
| | 9 | 8 | 9 | 8 | 7 | 5 | 4 | 3 |
| | 14 | 15 | 13 | 10 | 4 | 3 | 2 | 2 |
| | 15 | 15 | 12 | 12 | 12 | 10 | 8 | 7 |
| | 16 | 12 | 14 | 11 | 10 | 4 | 4 | 2 |
| | 17 | 7 | 8 | 7 | 7 | 5 | 4 | 3 |
| | 18 | 10 | 11 | 10 | 10 | 3 | 3 | 1 |
| moy | A | 7,9 | 9,8 | 14,5 | 17,8 | 20,8 | 21,5 | 17,1 |
| moy | B | 9,5 | 10,1 | 9,7 | 8,8 | 6,0 | 4,3 | 3,1 |
| SD | A | 3,3 | 3,6 | 4,6 | 9,1 | 7,7 | 4,9 | 5,1 |
| SD | B | 3,6 | 2,6 | 3,1 | 3,3 | 3,1 | 1,7 | 1,7 |
| *statistiques* | | *NS* | *NS* | *S* | *S* | *S* | *S* | *S* |
| % de réduction B/A | | / | -2,7% | 33,1% | 50,8% | 71,1% | 79,8% | 81,8% |

La population moyenne de puces hébergées par les chiens du groupe B est restée stable pendant le premier mois de l'étude puis a progressivement diminué pour atteindre 3,1 ± 1,7 au 5^{ème} mois. Dans le même temps et dans les mêmes conditions d'élevage et d'entretien, la population moyenne de puces du groupe A a constamment augmenté jusqu'au 4^{ème} mois (21,5 ± 4,9), pour fléchir ensuite sensiblement au 5^{ème} mois (17,1 ± 5,1). Les pourcentages de réduction du lot B par rapport au lot A sont de 33%, 51%, 71%, 80% et 82% respectivement à un, deux, trois, quatre et cinq mois après le début de l'étude.

L'analyse statistique n'a montré aucune différence significative à J0. Les moyennes des deux groupes sont significativement différentes entre J14 et J0+5 mois (p<0.05).

### 3.3 Examen cutané

Les examens cutanés ont globalement révélé une bonne qualité de la peau et du pelage, hormis un squamosis noté à 1/3 pour certains chiens en début et en cours d'étude :
J0 : chiens 12, 14, 15 et 21
J14 : chiens 12, 14, 15 et 21
J30 : chiens 12, 14, 15 et 21
J60 : chiens 2, 12, 15 et 21
J90 : chiens 2, 12, 15, 20 et 21
J120 : chiens 2, 12, 20 et 21
J150 : chiens 2, 12, 20 et 21

### 4 Conclusion

Les populations de puces chez des chiens infestés naturellement, avec un niveau d'infestation moyen en début d'étude (8 à 9.5 puces en moyenne) et vivant dans des chenils en terre battue sans traitement de l'environnement ont été suivies pendant 5 mois. Dans le lot alimenté avec l'aliment A, les populations ont progressé jusqu'à atteindre des moyennes supérieures à 20 puces par animal ; elles ont été significativement supérieures à celles des chiens alimentés avec l'aliment B qui ont progressivement diminué pour atteindre des moyennes inférieures à 5 puces par chien. La prise de l'aliment n'a entraîné aucun évènement indésirable.

A la fin de l'étude, le vétérinaire a été informé de la composition de l'aliment B, qui contenait la composition selon l'invention suivante :

| | |
|---|---|
| Extrait de Mélisse : | 37% |
| Extrait de Thym : | 25% |
| Extrait de Romarin: | 20% |
| Extrait d'Absinthe : | 15% |
| Extrait de Lemongrass : | 1% |

les pourcentages étant exprimés en masse par rapport à la masse totale de la composition, la composition étant utilisée à raison de 1% en masse par rapport à la masse de l'aliment à laquelle elle est ajoutée pour nourrir les animaux.

## Revendications

1. Composition consistant essentiellement enau moins un extrait de chacune des plantes aromatiques suivantes :
- les plantes aromatiques de la famille des *Lamiacées,*
- les plantes aromatiques de la famille des *Astéracées,* et
- les plantes aromatiques de la famille des *Poacées,*
- et éventuellement les plantes aromatiques de la famille des *Fabacées,*
ladite composition consistant en un mélange d'un extrait de mélisse, d'un extrait de thym, d'un extrait de romarin, d'un extrait d'absinthe et d'un extrait de lemongrass.

2. Composition selon la revendication 1, dans laquelle ledit au moins un extrait de *Lamiacées représente en masse* de 5 à 50%, ledit au moins un extrait *d'Astéracées* représente en masse de 1 à 20% et ledit au moins un extrait de *Poacées* représente en masse de 1 à 10%, et éventuellement ledit au moins un extrait de *Fabacées* représente en masse de 5 % à 15 %,
les pourcentages étant exprimés en masse par rapport à la masse totale de la composition.

3. Composition selon la revendication 1 ou 2, consistant en un mélange d'un extrait de mélisse, d'un extrait de thym, d'un extrait de romarin, d'un extrait d'absinthe, d'un extrait de lemongrass, et un extrait de fenugrec.

4. Composition selon la revendication 1 ou 2, consistant en :
- de 5 % à 50 % d'extrait de mélisse,
- de 10 % à 50 % d'extrait de thym,
- de 10 % à 50 % d'extrait de romarin,
- de 1 % à 20% d'extrait d'absinthe, et
- de 1 % à 10 % d'extrait de lemongrass,
les pourcentages étant exprimés en masse par rapport à la masse totale de la composition.

5. Composition selon la revendication 4, consistant en :
- de 5 % à 50 % d'extrait de mélisse,
- de 10 % à 50 % d'extrait de thym,
- de 10 % à 50 % d'extrait de romarin,
- de 1 % à 20% d'extrait d'absinthe,
- de 1 % à 10 % d'extrait de lemongrass, et
- de 5 % à 15 % d'extrait de fenugrec
les pourcentages étant exprimés en masse par rapport à la masse totale de la composition.

6. Composition selon l'une quelconque des revendications 1 à 5, comprenant en outre des vitamines, ou des acides aminés ou des minéraux, ou un mélange de ceux-ci.

7. Composition vétérinaire ou pharmaceutique comprenant à titre de substance active une composition selon l'une quelconque des revendications 1 à 6, en association avec un véhicule acceptable.

8. Aliment destiné à l'alimentation des animaux non humain comprenant de 0,01 % à 100 % d'une composition selon l'une quelconque des revendications 1 à 6, les pourcentages étant exprimés en masse par rapport à la masse totale de l'aliment.

9. Composition selon l'une quelconque des revendications 1 à 6 en tant que médicament.

10. Composition selon l'une quelconque des revendications 1 à 6 ou selon la revendication 9, pour son utilisation dans le cadre du traitement des mammifères domestiques pour la prévention ou le traitement contre l'infestation par des puces ou pulicose, et de la prévention des symptômes associés.

11. Procédé de préparation d'une composition selon l'une quelconque des revendications 1 à 6, dans lequel :
- on tasse des feuilles mélisse, de thym, de romarin, d'absinthe et de lemongrass, dans un volume d'eau suffisant pour les recouvrir, pour obtenir un mélange de feuilles tassées,
- on laisse macérer pendant au moins deux semaines à température ambiantes ledit mélange de feuilles tassées, en mélangeant régulièrement ledit mélange de feuilles tassées,
- on élimine le macérat pour conserver la fraction liquide.
- et éventuellement on place sous agitation mécanique la fraction liquide pour obtenir une composition homogène.

## Patentansprüche

1. Zusammensetzung, die im Wesentlichen aus mindestens einem Extrakt jeder der folgenden Gewürzpflanzen besteht:
- Gewürzpflanzen der Familie der *Lippenblütler,*
- Gewürzpflanzen der Familie der *Korbblütler,* und
- Gewürzpflanzen der Familie der *Süßgräser,*
- und optional Gewürzpflanzen der Familie der *Hülsenfrüchtler,* wobei die Zusammensetzung aus einer Mischung aus einem Zitronenmelissenextrakt, einem Thymianextrakt, einem Rosmarinextrakt, einem Wermutextrakt und einem Zitronengrasextrakt besteht.

2. Zusammensetzung nach Anspruch 1, wobei der mindestens eine Extrakt der *Lippenblütler* eine Masse von 5 bis 50 % ausmacht, der mindestens eine Extrakt der *Korbblütler* eine Masse von 1 bis 20 % ausmacht und der mindestens eine Extrakt der *Süßgräser* eine Masse von 1 bis 10 % ausmacht und optional der mindestens eine Extrakt der *Hülsenfrüchtler* eine Masse von 5 % bis 15 % ausmacht,
wobei die Prozentsätze in Masse relativ zu der Gesamtmasse der Zusammensetzung ausgedrückt sind.

3. Zusammensetzung nach Anspruch 1 oder 2, bestehend aus einer Mischung aus einem Zitronenmelissenextrakt, einem Thymianextrakt, einem Rosmarinextrakt, einem Wermutextrakt, einem Zitronengrasextrakt und einem Bockshornkleeextrakt.

4. Zusammensetzung nach Anspruch 1 oder 2, bestehend aus:
- 5 % bis 50 % Zitronenmelissenextrakt,
- 10 % bis 50 % Thymianextrakt,
- 10 % bis 50 % Rosmarinextrakt,
- 1 % bis 20 % Wermutextrakt, und
- 1 % bis 10 % Zitronengrasextrakt,
wobei die Prozentsätze in Masse relativ zu der Gesamtmasse der Zusammensetzung ausgedrückt sind.

5. Zusammensetzung nach Anspruch 4, bestehend aus:
- 5 % bis 50 % Zitronenmelissenextrakt,
- 10 % bis 50 % Thymianextrakt,
- 10 % bis 50 % Rosmarinextrakt,
- 1 % bis 20 % Wermutextrakt,
- 1 % bis 10 % Zitronengras-Extrakt, und
- 5 % bis 15 % Bockshornkleeextrakt
wobei die Prozentsätze in Masse relativ zu der Gesamtmasse der Zusammensetzung ausgedrückt sind.

6. Zusammensetzung nach einem der Ansprüche 1 bis 5, umfassend ferner Vitamine oder Aminosäuren oder Mineralien oder eine Mischung davon.

7. Veterinärmedizinische oder pharmazeutische Zusammensetzung, umfassend als Wirkstoff eine Zusammensetzung nach einem der Ansprüche 1 bis 6 in Verbindung mit einem akzeptablen Träger.

8. Nahrung, die zum Ernähren nicht menschlicher Tiere bestimmt ist, umfassend und 0,01 % bis 100 % einer Zusammensetzung nach einem der Ansprüche 1 bis 6, wobei die Prozentsätze in Masse relativ zu der Gesamtmasse der Nahrung ausgedrückt sind.

9. Zusammensetzung nach einem der Ansprüche 1 bis 6 als Arzneimittel.

10. Zusammensetzung nach einem der Ansprüche 1 bis 6 oder nach Anspruch 9 zur Verwendung im Rahmen der Behandlung von Haussäugetieren für die Vorbeugung oder die Behandlung eines Befalls durch Flöhe oder Pulikose und für die Vorbeugung der damit verbundenen Symptome.

11. Verfahren zum Herstellen einer Zusammensetzung nach einem der Ansprüche 1 bis 6, wobei:
- Zitronenmelisse-, Thymian-, Rosmarin-, Wermut- und Zitronengrasblätter in einer Wassermenge, die zum Bedecken davon ausreicht, zum Erhalten einer Mischung aus zusammengepressten Blättern zusammengepresst werden,
- die Mischung aus zusammengepressten Blättern mindestens zwei Wochen lang bei Umgebungstemperatur mazerieren gelassen wird, wobei die Mischung aus zusammengepressten Blättern regelmäßig gemischt wird,
- das Mazerat zum Konservieren des flüssigen Anteils entfernt wird,
- und optional der flüssige Anteil zum Erhalten einer homogenen Zusammensetzung mechanisch gerührt wird.

## Claims

1. A composition consisting essentially of an extract of each of the following aromatic plants:
aromatic plants of the *Lamiaceae* family,
aromatic plants of the *Asteraceae* family, and
aromatic plants of the *Poaceae* family,
and eventually aromatic plants of the *Fabaceae* family
said composition consisting of a mixture of a lemon balm extract, a thyme extract, a rosemary extract, a wormwood extract and a lemongrass extract.

2. The composition according to claim 1, wherein the extract of aromatic plants of the *Lamiaceae* family represents from 5 to 50% by mass, the extract of aromatic plants of the *Asteraceae* family represents from 1 to 20% by mass, and the extract of aromatic plants of the *Poaceae* family represents from 1 to 10% by mass, and eventually the extract of aromatic plants of the *Fabaceae* family represents from 5% to 15% by mass, the percentages being expressed by mass relative to the total mass of the composition.

3. The composition according to claim 1 or 2, Consisting in a mixture of a mixture of a lemon balm extract, a thyme extract, a rosemary extract, a wormwood extract, a lemongrass extract and a fenugreek extract.

4. The composition according to claim 1 or 2, consisting of:
from 5% to 50% lemon balm extract,
from 10% to 50% thyme extract,
from 10% to 50% rosemary extract,
from 1% to 20% wormwood extract, and
from 1% to 10% lemongrass extract,
the percentages being expressed by mass relative to the total mass of the composition.

5. The composition according to claim 4, consisting of:
from 5% to 50% lemon balm extract,
from 10% to 50% thyme extract,
from 10% to 50% rosemary extract,
from 1% to 20% wormwood extract, and
from 1% to 10% lemongrass extract,
from 5% to 15% fenugreek extract
the percentages being expressed by mass relative to the total mass of the composition.

6. The composition according to anyone of claims 1 to 5, further comprising vitamins, or amino acids or minerals, or a mixture thereof.

7. A veterinary or pharmaceutical composition comprising, as an active substance, a composition according to anyone of claims 1 to 6, in association with an acceptable carrier.

8. A food intended for feeding non-human animals comprising from 0.01% to 100% of a composition according to anyone of claims 1 to 6, the percentages being expressed by mass relative to the total mass of the food.

9. A composition according to anyone of claims 1 to 6, as a drug.

10. A composition according to anyone of claims 1 to 6, or according to claim 9, for its use for the treatment of domestic mammals for preventing or treating flea infestation or pulicosis, and the prevention of associated symptoms.

11. A process for preparing a composition according to anyone of claims 1 to 6, wherein:
- lemon balm, thyme, rosemary, wormwood and lemongrass leaves are compacted in a sufficient volume of water to cover them, to obtain a mixture of compacted leaves,
- the mixture of compacted leaves is left to macerate for at least two weeks at room temperature, with regular mixing of the mixture of compacted leaves, to obtain a liquid fraction and a macerate,
- the macerate is eliminated to preserve the liquid fraction, and
- the liquid fraction is mechanically stirred to obtain a homogeneous composition.
